# EUROPEAN PATENT SPECIFICATION

(11) **EP 2 224 934 B1**
(45) Date of publication and mention of the grant of the patent: **15.08.2012**
(21) Application number: 08864978.5
(22) Date of filing: 19.12.2008
(51) Int. Cl.: A61K 35/32, C12N 5/077

(54) **Use of osteoblasts in the treatment of inflammatory rheumatic diseases**
Verwendung von Osteoblasten in der Behandlung entzündlich rheumatischer Erkrankungen
Utilisation d'ostéoblastes dans le traitement de maladies rhumatismales inflammatoires

(30) Priority: 21.12.2007 EP 07150368
(43) Date of publication of application: 08.09.2010
(73) Proprietor: Bone Therapeutics S.A., 6041 Gosselies (BE)
(72) Inventor: BASTIANELLI, Enrico, B-1640 Rhode St Genese (BE); BADOER, Cindy, B-1600 Sint-Pieters-Leeuw (BE); BIZIMUNGU, Christelle, B-1404 Bornival (BE); PESESSE, Xavier, B-1430 Bierghes (BE)
(74) Representative: Michalík, Andrej
(86) International application number: PCT/EP2008/068007
(87) International publication number: WO 2009/080749

(56) References cited:
- WO-A-02/04606
- WO-A-2005/089127
- AUGELLO ANDREA ET AL: "Cell therapy using allogeneic bone marrow mesenchymal stem cells prevents tissue damage in collagen-induced arthritis" ARTHRITIS & RHEUMATISM, vol. 56, no. 4, April 2007 (2007-04), pages 1175-1186, XP002517050 ISSN: 0004-3591
- GANGJI ET AL: "Treatment of osteonecrosis of the femoral head with implantation of preosteoblastic cells into the necrotic zone: Case report" BONE, PERGAMON PRESS., OXFORD, GB, vol. 40, no. 6, 1 June 2007 (2007-06-01), page S47, XP022121665 ISSN: 8756-3282
- NIEMEYER PHILIPP ET AL: "Comparison of immunological properties of bone marrow stromal cells and adipose tissue-derived stem cells before and after osteogenic differentiation in vitro" TISSUE ENGINEERING, LARCHMONT, NY, US, vol. 13, no. 1, 1 January 2007 (2007-01-01), pages 111-121, XP002496955 ISSN: 1076-3279
- CASTANO-IZQUIERDO HAROLD ET AL: "Pre-culture period of mesenchymal stem cells in osteogenic media influences their in vivo bone forming potential" JOURNAL OF BIOMEDICAL MATERIALS RESEARCH, vol. 82A, no. 1, July 2007 (2007-07), pages 129-138, XP002517051 ISSN: 1549-3296(print) 1552-4965(ele
- NAUTA ALMA J ET AL: "Donor-derived mesenchymal stem cells are immunogenic in an allogeneic host and stimulate donor graft rejection in a nonmyeloablative setting" BLOOD, vol. 108, no. 6, September 2006 (2006-09), pages 2114-2120, XP002517052 ISSN: 0006-4971
- ZHENG Z H ET AL: "Allogeneic mesenchymal stem cell and mesenchymal stem cell-differentiated chondrocyte suppress the responses of type II collagen-reactive T cells in rheumatoid arthritis." RHEUMATOLOGY (OXFORD, ENGLAND) JAN 2008, vol. 47, no. 1, January 2008 (2008-01), pages 22-30, XP002517133 ISSN: 1462-0332

## Description

### Field of the Invention

The invention relates to therapeutic applications of bone-forming cells in the treatment of inflammatory rheumatic diseases (IRD), and in particular in the treatment of inflammation in (inflammatory component of) IRD.

### Background to the Invention

Rheumatic diseases encompass a variety of painful disorders which affect the loco-motor system particularly including joints, muscles, connective tissues, soft tissues around the joints and bones.

Inflammation and/or autoimmune reactions contribute to the aetiology of many rheumatic diseases. Such conditions, commonly referred to as inflammatory rheumatic diseases or IRD, include without limitation arthritis of various origins, osteoarthritis and so forth.

Presently available treatments for IRD mainly include disease-modifying antirheumatic drugs (DMARD), glucocorticoids, non-steroidal anti-inflammatory drugs (NSAID) and analgesics.

Accordingly, there exists a need for further treatment modalities in IRD, and in particular for treatment modalities targeting the inflammatory component of IRD.

WO 2005/089127 includes osteogenic cells in a scaffold apparatus to regenerate osteochondral interfaces in osteoarthritis. WO 2007/093431 suggests to use osteoblasts for the treatment of rheumatoid arthritis and osteonecrosis. These documents do not disclose the anti-inflammatory action of bone-forming cells, and do not disclose the use of bone-forming cells to suppress the inflammatory component of rheumatic diseases.

Liu et al. 2006 (J Immunol 176(5): 2864-71) discloses immuoprivileged and immunomodulatory properties of osteogenic cells differentiated from mesenchymal stem cells in the context of allogeneic tissue transplantation. However, the mechanisms of tissue rejection in allogeneic transplantation are evidently different from the mechanisms underlying inflammation in rheumatic diseases. This is reflected *inter alia* in the distinct groups of drugs that are currently used to treat these conditions. Consequently, Liu *et al*. 2006 does not disclose any anti-inflammatory actions of bone-forming cells, and does not disclose the use of bone-forming cells to suppress the inflammatory component of rheumatic diseases.

### Description of the Invention

The present inventors surprisingly realised that bone-forming cells in particular osteoblasts display potent immunosuppressive and specifically anti-inflammatory actions, in addition to expected osteoregenerative actions, and are thus particularly useful in the treatment of inflammatory rheumatic diseases (IRD) in subjects, and more specifically for the treatment of inflammation in, i.e., the inflammatory component of, IRD in subjects.

The present invention provides subject-matter as set forth in any one and all of (i) to (x) below:
(i) Isolated osteoblasts for use in treating the inflammatory component of inflammatory rheumatic diseases (IRD).
(ii) Isolated osteoblasts for use as set forth in (i) above, wherein said osteoblasts are derived by differentiation from isolated bone marrow stem cells or mesenchymal stem cells.
(iii) Isolated osteoblasts for use as set forth in (i) or (ii) above wherein the osteoblasts are of human origin and the medicament is to be employed for autologous or allogeneic administration to human subjects.
(iv) Isolated osteoblasts for use as set forth in (iii) above wherein said administration is systemic, topical, intra-articular or peri-articular.
(v) Isolated osteoblasts for use as set forth in any one of (i) to (iv) above wherein the IRD is chosen from osteoarthritis (OA), psoriatic arthropathy, gout, pseudogout and arthritis of various origins comprising rheumatoid arthritis (RA), enteropathic arthritis, reactive arthritis and Reiter syndrome, osteonecrosis, pauciarticular juvenile rheumatoid arthritis, Still disease, Behçet disease, systemic lupus erythematosus, septic arthritis and spondyloarthropathies comprising ankylosing spondylitis, enteropathic spondylitis and undifferentiated spondyloarthropathy.
(vi) Isolated osteoblasts for use as set forth in any one of (i) to (iv) above wherein the IRD is other than osteoarthritis (OA), rheumatoid arthritis (RA) and osteonecrosis.
(vii) Isolated osteoblasts as set forth in any one of (i) to (vi) above wherein the IRD comprises both an inflammation and bone lesion(s).
(viii) Isolated osteoblasts as set forth in any one of (i) to (vi) above wherein the IRD comprises an inflammation and does not comprise bone lesion(s).
(ix) A pharmaceutical composition comprising osteoblasts and an agent chosen from disease-modifying antirheumatic drugs (DMARD) and glucocorticoids, for simultaneous, sequential or separate use in treating the inflammatory component of IRD.
(x) A pharmaceutical composition comprising osteoblasts for use in treating the inflammatory component of IRD, particularly for use as set forth in any one of (ii) to (viii) above.

The subject-matter provided by the invention thus specifically belongs to the disclosure, description and teaching of the present specification.

The specification further describes isolated bone-forming cells in particular isolated osteoblasts for use in treating IRD, as well as the use of isolated bone-forming cells in particular isolated osteoblasts for the manufacture of a medicament for the treatment of IRD. Further disclosed are isolated bone-forming cells in particular isolated osteoblasts for use in treating inflammation in (the inflammatory component of) IRD, as well as the use of isolated bone-forming cells in particular isolated osteoblasts for the manufacture of a medicament for treating inflammation in (the inflammatory component of) IRD.

The specification also relates to a method for preventing and/or treating IRD in a subject in need of such treatment, comprising administering to said subject a prophylactically or therapeutically effective amount of isolated bone-forming cells in particular isolated osteoblasts. Also disclosed is a method for preventing and/or treating inflammation in (the inflammatory component of) IRD in a subject in need of such treatment, comprising administering to said subject a prophylactically or therapeutically effective amount of isolated bone-forming cells in particular isolated osteoblasts. Also, specification describes a pharmaceutical composition comprising isolated bone-forming cells in particular isolated osteoblasts for use in treating IRD. Also disclosed is a pharmaceutical composition comprising isolated bone-forming cells in particular isolated osteoblasts for use in treating inflammation in (the inflammatory component of) IRD.

Also described are isolated bone-forming cells in particular isolated osteoblasts for use in treating inflammation, as well as the use of isolated bone-forming cells in particular isolated osteoblasts for the manufacture of a medicament for the treatment of inflammation. As well described is a method for preventing and/or treating inflammation in a subject in need of such treatment, comprising administering to said subject a prophylactically or therapeutically effective amount of isolated bone-forming cells in particular isolated osteoblasts. Further described is a pharmaceutical composition comprising isolated bone-forming cells in particular isolated osteoblasts for use in treating inflammation.

The term "isolated" as used herein in relation to cells or cell populations implies that such cells or cell populations do not form part of an animal or human body, but are removed or separated there from.

Said bone-forming cells in particular osteoblasts may preferably be of mammal origin including non-human mammal origin and more preferably are of human origin. The bone-forming cells in particular osteoblasts may be usually obtained from or derived from a biological sample of a subject (*i.e*., a sample removed from a subject and comprising cells thereof) such as preferably a human or non-human mammal subject.

Subjects preferably encompass warm-blooded animals, more preferably mammal subjects, including human and non-human mammal subjects, even more preferably primate subjects, including human and non-human primate subjects, and yet more preferably human subjects. The bone-forming cells in particular osteoblasts may thus also be of such origins.

Said bone-forming cells in particular osteoblasts may be preferably employed for autologous administration (*i.e*., administered to the same subject from which the cells have been obtained or derived) or allogeneic administration (*i.e*., administered to a subject other than, but of the same species as, the subject from which the cells have been obtained or derived). Also possible may be xenogenic administration of said bone-forming cells in particular osteoblasts (*i.e*., wherein cells obtained or derived from a subject of one species are administered to a subject of a different species).

Preferably herein, human bone-forming cells in particular osteoblasts are to be employed for autologous or allogeneic administration to human subjects having IRD. Autologous administration may be particularly preferred.

The term "bone-forming cells" as used herein generally refers to cells capable of contributing to, or capable of developing to cells which can contribute to, the formation of bone material and/or bone matrix, and particularly denotes isolated cells or cell populations which a) are capable of undergoing osteogenic differentiation, or b) are committed towards osteogenic differentiation, or c) have at least partly progressed along osteogenic differentiation, more preferably denotes isolated cells or cell populations listed under any of b) or c). Without limitation, bone-forming cells particularly encompass osteoprogenitors, osteoblasts, osteocytes and other cell types of the osteogenic lineage as known in the art.

A skilled person thus generally appreciates the bounds of the term "bone-forming cells" as intended herein. Nevertheless, by means of further guidance and not limitation the present bone-forming cells may display any one, more or all following characteristics:
a) the cells comprise expression of alkaline phosphatase (ALP), more specifically ALP of the bone-liver-kidney type, or expression of osteocalcin or both;
b) optionally, the cells comprise expression of any one or more of procollagen type 1 amino-terminal propeptide (P1 NP), osteonectin (ON), osteopontin (OP) and bone sialoprotein (BSP);
c) optionally, the cells comprise expression of any one or more mesenchymal markers CD105, CD73 and CD90;
d) the cells show evidence of ability to mineralize the external surroundings, or synthesize calcium-containing extracellular matrix (*e.g*., when exposed to osteogenic medium; see Jaiswal et al. 1997. J Cell Biochem 64: 295-312). Calcium accumulation inside cells and deposition into matrix proteins can be conventionally measured for example by culturing in ⁴⁵Ca²⁺, washing and re-culturing, and then determining any radioactivity present inside the cell or deposited into the extracellular matrix (U.S. Pat. No. 5,972,703), or by assaying culture substrate for mineralization using a Ca²⁺ assay kit (Sigma Kit #587), or as described in the examples;
e) the cells substantially do not differentiate towards any one of, and preferably towards neither of cells of adipocytic lineage (*e.g*., adipocytes) or chondrocytic lineage (*e.g*., chondrocytes). The absence of differentiation towards such cell lineages may be tested using standard differentiation inducing conditions established in the art (*e.g*., see Pittenger et al. 1999. Science 284: 143-7), and assaying methods (*e.g*., when induced, adipocytes typically stain with oil red O showing lipid accumulation; chondrocytes typically stain with alcian blue or safranin O). Substantially lacking propensity towards adipogenic and/or chondrogenic differentiation may typically mean that less than 50%, or less than 30%, or less than 5%, or less than 1% of the tested cells would show signs of adipogenic or chondrogenic differentiation when applied to the respective test.

In an option the bone-forming cells in particular the osteoblasts may display all characteristics listed under a), d) and e) above.

Wherein a cell is said to be positive for a particular component (*e.g*., marker or enzyme), this means that a skilled person will conclude the presence or evidence of a distinct signal, e.g., antibody-detectable or detection by reverse transcription polymerase chain reaction, for that component when carrying out the appropriate measurement, compared to suitable controls. Where the method allows for quantitative assessment of the component, positive cells may on average generate a signal that is significantly different from the control, e.g., but without limitation, at least 1.5-fold higher than such signal generated by control cells, e.g., at least 2-fold, at least 4-fold, at least 10-fold, at least 20-fold, at least 30-fold, at least 40-fold, at least 50-fold higher or even higher.

The expression of the above cell-specific markers can be detected using any suitable immunological technique known in the art, such as immuno-cytochemistry or affinity adsorption, Western blot analysis, FACS, ELISA, *etc*., or by any suitable biochemical assay of enzyme activity (*e.g*., for ALP), or by any suitable technique of measuring the quantity of the marker mRNA, *e.g*., Northern blot, semi-quantitative or quantitative RT-PCR, *etc*. Sequence data for markers listed in this disclosure are known and can be obtained from public databases such as GenBank (http://www.ncbi.nlm.nih.gov/).

Isolated bone-forming cells in particular isolated osteoblasts or cell populations for use in the invention and in the specification may be obtained or derived in any suitable manner known in the art. For example bone-forming cells or cell derived in any suitable manner known in the art. For example, bone-forming cells or cell populations as described herein, or in an embodiment osteoblasts may be derived by differentiation from relatively less differentiated adult progenitors or stem cells, such as, *e.g*., from mesenchymal stem cells, using differentiation protocols known *per se.* Without limitation, one suitable method to obtain bone-forming osteoblasts has been disclosed in WO 2007/093431 and involves culturing isolated bone marrow stem cells (BMSC) or mesenchymal stem cells (MSC) in the presence of plasma and basic fibroblast growth factor (FGF-2). In another example, osteogenic lineage cells may be obtained by differentiating MSC in osteogenic medium as described by Pittenger et al. 1999 (Science 284: 143-7) and Jaiswal et al. 1997 (*supra*)*.* In another example bone-forming cells or cell populations as described herein, or in an embodiment osteoblasts may be isolated and optionally cultured and/or expanded from biological samples comprising such cells. For example, osteoblasts can be directly isolated and cultured from trabecular bone as described by Skjodt et al. 1985 (J Endocrinol 105: 391-6).

The term "inflammatory rheumatic disease" or "IRD" as used herein generally includes all rheumatic diseases which entail an inflammatory and/or autoimmunity component, and particularly which entail at least an inflammatory component. By means of example and not limitation, IRD particularly comprises osteoarthritis (OA), psoriatic arthropathy, gout, pseudogout and arthritis of various origins including among others rheumatoid arthritis (RA), enteropathic arthritis, reactive arthritis and Reiter syndrome, osteonecrosis, pauciarticular juvenile rheumatoid arthritis, Still disease, Behçet disease, systemic lupus erythematosus, septic arthritis and spondyloarthropathies such as *inter alia* ankylosing spondylitis and enteropathic spondylitis and undifferentiated spondyloarthropathy.

Hence, in an embodiment the disclosure may relate to any one IRD chosen from osteoarthritis (OA), psoriatic arthropathy, gout, pseudogout and arthritis of various origins including among others rheumatoid arthritis (RA), enteropathic arthritis, reactive arthritis and Reiter syndrome, osteonecrosis, pauciarticular juvenile rheumatoid arthritis, Still disease, Behçet disease, systemic lupus erythematosus, septic arthritis and spondyloarthropathies such as *inter alia* ankylosing spondylitis and enteropathic spondylitis and undifferentiated spondyloarthropathy.

In another embodiment, the disclosure may relate to any one IRD other than osteoarthritis (OA), osteonecrosis and rheumatoid arthritis (RA).

In yet another embodiment, the disclosure may relate to any one IRD chosen from psoriatic arthropathy, gout, pseudogout and arthritis of various origins including among others enteropathic arthritis, reactive arthritis and Reiter syndrome, pauciarticular juvenile rheumatoid arthritis, Still disease, Behçet disease, systemic lupus erythematosus, septic arthritis and spondyloarthropathies such as *inter alia* ankylosing spondylitis and enteropathic spondylitis and undifferentiated spondyloarthropathy.

The present bone-forming cells in particular osteoblasts may be particularly useful in treating IRD diseases (or treating inflammation in or inflammatory component of said diseases), which comprise both inflammation, with or without an autoimmunity component, and bone lesion(s) such as, for example, erosion or subchondral lesions. Herein, the bone-forming cells in particular in this embodiment osteoblasts can synergically ameliorate both said pathologies, whereby a more pronounced therapeutic improvement can be achieved

In another option, the bone-forming cells in particular in an embodiment osteoblasts may be used in treating IRD diseases (or treating the inflammation in or inflammatory component of said diseases), wherein said diseases include inflammation and do not include bone lesion(s). By means of example, a IRD disease may be treated in a patient wherein the inflammation component is present, and wherein bone lesion(s) has not yet ensued. The use of bone-forming cells in particular osteoblasts in such diseases would not have been previously indicated, since prior to the present disclosure of the anti-inflammatory effects of bone-forming cell in particular osteoblasts, there would be no expectation of any benefits in such diseases from administration of bone-forming cells in particular osteoblasts.

Prophylaxis and/or treatment of inflammation in, *i.e.*, of the inflammatory component of, IRD, may in particular involve suppressing, reducing or decreasing the level or degree of systemic and/or local inflammation in said IRD, *i.e*., suppressing, reducing or decreasing the inflammation (inflammatory component). Said level or degree of inflammation can be suitably assessed as known *per se,* for example and without limitation by measuring symptoms of inflammation (*e.g*., fever, tissue swelling, pain, loss of function, *etc*.) and/or by measuring cellular and/or molecular markers of inflammation, such as, for example, IL-1α, IL-1β, IL-2, IL-6 IL-8, and TNFα, *e.g*., local and/or systemic levels of said markers.

The isolated bone-forming cells to be employed in the present specification in particular osteoblasts to the employed in the present invention can be suitably formulated into and administered as pharmaceutical compositions.

Such pharmaceutical compositions may comprise, in addition to the bone-forming cells as described herein, in particular the osteoblasts as defined herein, a pharmaceutically acceptable excipient, carrier, buffer, preservative, stabiliser, anti-oxidant or other material well known to those skilled in the art. Such materials should be non-toxic and should not interfere with the activity of the cells. The precise nature of the carrier or other material will depend on the route of administration. For example, the composition may be in the form of a parenterally acceptable aqueous solution, which is pyrogen-free and has suitable pH, isotonicity and stability. For general principles in medicinal formulation, the reader is referred to Cell Therapy: Stem Cell Transplantation, Gene Therapy, and Cellular Immunotherapy, by G. Morstyn & W. Sheridan eds., Cambridge University Press, 1996; and Hematopoietic Stem Cell Therapy, E. D. Ball, J. Lister & P. Law, Churchill Livingstone, 2000.

Such pharmaceutical compositions may contain further components ensuring the viability of the cells therein. For example, the compositions may comprise a suitable buffer system (e.g., phosphate or carbonate buffer system) to achieve desirable pH, more usually near neutral pH, and may comprise sufficient salt to ensure isoosmotic conditions for the cells to prevent osmotic stress. For example, suitable solution for these purposes may be phosphate-buffered saline (PBS), sodium chloride solution, Ringer's Injection or Lactated Ringer's Injection, as known in the art. Further, the composition may comprise a carrier protein, *e.g*., albumin, which may increase the viability of the cells.

The pharmaceutical compositions may comprise further components useful in the repair of bone wounds and defects. For example, such components may include without limitation hydroxyapatite/tricalcium phosphate particles (HA/TCP), gelatine, poly-lactic acid, poly-lactic glycolic acid, hyaluronic acid, chitosan, poly-L-lysine, and collagen. For example, the bone-forming cells in particular osteoblasts may be combined with demineralised bone matrix (DBM) or other matrices to make the composite osteogenic (bone forming in it own right) as well as osteo-inductive. Similar methods using autologous bone marrow cells with allogeneic DBM have yielded good results (Connolly et al. 1995. Clin Orthop 313: 8-18).

The pharmaceutical composition can further include or be co-administered with a complementary bioactive factor such as a bone morphogenetic protein, such as BMP-2 or BMP-4, BMP-7 or any other growth factor. Other potential accompanying components include inorganic sources of calcium or phosphate suitable for assisting bone regeneration (WO 00/07639). If desired, cell preparation can be administered on a carrier matrix or material to provide improved tissue regeneration. For example, the material can be a granular ceramic, or a biopolymer such as gelatine, collagen, osteonectin, fibrinogen, or osteocalcin. Porous matrices can be synthesized according to standard techniques (*e.g.*, Mikos et al., Biomaterials 14:323, 1993; Mikos et al., Polymer 35:1068, 1994; Cook et al., J. Biomed. Mater. Res. 35:513, 1997).

The pharmaceutical compositions can further include or be co-administered in combination with any art-known therapies useful in IRD, such as without limitation with disease-modifying antirheumatic drugs (DMARD), glucocorticoids, non-steroidal anti-inflammatory drugs (NSAID) or analgesics. Hence, the specification also provides a pharmaceutical composition comprising bone-forming cells in particular osteoblasts and an agent chosen from DMARD, glucocorticoids, NSAID and analgesics for simultaneous, sequential or separate use in treating IRD.

The bone-forming cells in particular osteoblasts are to be administered in a "prophylactically effective amount" (*i.e.*, an amount of that inhibits or delays in a subject the onset of a disorder as being sought by a researcher, veterinarian, medical doctor or other clinician) or in a "therapeutically effective amount" (*i.e*., an amount that elicits the biological or medicinal response in a subject that is being sought by a researcher, veterinarian, medical doctor or other clinician, which may include *inter alia* alleviation of the symptoms of the disease or disorder being treated). The dosage or amount of bone-forming cells used, in particular osteoblasts optionally in combination with one or more other active agents, depends on the individual case and is, as is customary, to be adapted to the individual circumstances to achieve an optimum effect. Thus, it depends on the nature and the severity of the disorder to be treated, and also on the sex, age, body weight, general health, diet, mode and time of administration, and individual responsiveness of the subject to be treated, on the route of administration, efficacy, stability and duration of action, on whether the therapy is acute or chronic or prophylactic, or on whether other active compounds are administered in addition to the bone-forming cells as taught herein, in particular the osteoblasts of the invention.

By means of example and not limitation, a dose of between about 1x10³ and about 1x10⁹ bone forming cells in particular osteoblasts, or between about 1x10⁴ and about 1x10⁸ bone forming cells in particular osteoblasts, or between about 1x10⁵ and about 1x10⁷ bone forming cells in particular osteoblasts, or between about 1x10⁶ and 1x10⁸ bone forming cells in particular osteoblasts, may be administered, locally and/or systemically, to a subject, preferably a non-human mammal or human subject. Such administration may be one-time or repeated, or may be done by unit of volume. By means of example and not limitation, frequency of repeated administration may be once or twice per day; once, twice or more times per week; or once, twice or more times per month.

The specification further also encompasses methods of producing said pharmaceutical The further also encompasses methods of producing said pharmaceutical compositions, wherein said pharmaceutical composition are intended for use in treating IRD, by admixing bone-forming cells as disclosed herein in particular osteoblasts as defined herein with one or more additional components as above.

The bone-forming cells in particular osteoblasts or pharmaceutical formulations comprising such can be administered in a manner that permits them to graft or migrate to the intended tissue site and reconstitute or regenerate the functionally deficient area. Administration of the composition will depend on the musculoskeletal site being repaired. For example, administration may occur by injection or implantation directly into intra-articular cavity in case of disorders of joints. In other circumstances, the bone-forming cells in particular osteoblasts or pharmaceutical formulations comprising such may be administered systemically, whereby their anti-inflammatory actions may occur systemically or they may migrate to diseased areas. Hence, in general examples, the administration may be *inter alia* systemic, topical, intra-articular or peri-articular.

Hence, in an option the pharmaceutical cell preparation as define above may be administered in a form of liquid composition.

In another option, the bone-forming cells in particular osteoblasts or cell populations may be transferred to and/or cultured on suitable substrate to provide for implants. The substrate on which the cells can be applied and cultured can be a metal, such as titanium, cobalt/chromium alloy or stainless steel, a bioactive surface such as a calcium phosphate, polymer surfaces such as polyethylene, and the like. Although less preferred, siliceous material such as glass ceramics, can also be used as a substrate. Most preferred are metals, such as titanium, and calcium phosphates, even though calcium phosphate is not an indispensable component of the substrate. The substrate may be porous or non-porous.

For example, cells that have proliferated, or that are being differentiated in culture dishes, can be transferred onto three-dimensional solid supports in order to cause them to multiply and/or continue the differentiation process by incubating the solid support in a liquid nutrient medium of the invention, if necessary. Cells can be transferred onto a three-dimensional solid support, *e.g.* by impregnating said support with a liquid suspension containing said cells. The impregnated supports obtained in this way can be implanted in a human subject. Such impregnated supports can also be re-cultured by immersing them in a liquid culture medium, prior to being finally implanted.

The three-dimensional solid support needs to be biocompatible so as to enable it to be implanted in a human. It can be of any suitable shape such as a cylinder, a sphere, a plate, or a part of arbitrary shape. Of the materials suitable for the biocompatible three-dimensional solid support, particular mention can be made of calcium carbonate, and in particular aragonite, specifically in the form of coral skeleton, porous ceramics based on alumina, on zirconia, on tricalcium phosphate, and/or hydroxyapatite, imitation coral skeleton obtained by hydrothermal exchange enabling calcium carbonate to be transformed into hydroxyapatite, or else apatite-wollastonite glass ceramics, bioactive glass ceramics such as Bioglass(TM) glasses.

### General definitions

Unless otherwise defined, all terms used in disclosing the invention, including technical and scientific terms, have the meaning as commonly understood by one of ordinary skill in the art to which this invention belongs.

As used herein, the singular forms "a", "an", and "the" include both singular and plural referents unless the context clearly dictates otherwise. By way of example, "a cell" refers to one or more than one cell.

The terms "comprising", "comprises" and "comprised of" as used herein are synonymous with "including", "includes" or "containing", "contains", and are inclusive or open-ended and do not exclude additional, non-recited members, elements or method steps.

The recitation of numerical ranges by endpoints includes all numbers and fractions subsumed within that range, as well as the recited endpoints.

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

All documents cited in the present specification are hereby incorporated by reference in their entirety. In particular, the teachings of all documents herein specifically referred to are incorporated by reference.

### Brief Description of the Figures

**Figure 1** shows mesenchymal and bone markers expression by bone-forming cells.
**Figure 2** shows mineralization (A) and ALP (B) staining by bone-forming cells.
**Figure 3** shows comparison between Ankles Diameters in CARRA treated animals (light grey), CARRA+DEX treated animals (dark grey), CARRA+OB treated animals (dark) and CARRA+OB+DEX treated animals (dotted line). Diameters reported as % increase versus baseline.
**Figure 4** shows comparison between Paw Diameters in CARRA treated animals (diamonds) CARRA+DEX treated animals (squares), and CARRA+OB treated animals (triangles). Diameters reported as % increase versus baseline.
**Figure 5** shows comparison between Paw Diameters in ADJ treated animals (diamonds), ADJ+DEX treated animals (squares), and ADJ+OB treated animals (triangles). Diameters reported as % increase versus baseline.

Abbreviations used in figures are as follows: CARRA: Carrageenan 0.7%; ADJ: Complete Freund adjuvant 75µg; DEX: dexamethasone 1 mg/kg; OB: osteoblasts 1*10⁶

### EXAMPLE 1

### Experimental procedures

In the following, procedures are described leading to derivation of bone-forming cells either (A) from bone marrow stem cells (BMSC) substantially as described in WO 2007/093431, or (B) by further differentiating the cells of (A) in osteogenic medium, or (C) by expanding osteoblasts from trabecular bone.

### A. Osteoblast derivation from BMSC

20 to 60ml of heparinized bone marrow (BM) were obtained from iliac crest of patients suffering from bone diseases. BM was mixed with phosphate-buffered saline (PBS, 2v:v) and layered on density gradient Ficoll solution. After centrifugation, mononuclear cells were harvested from the interface and washed twice in PBS. In parallel, serum from patients or healthy donors was obtained after centrifugation of 160 ml of blood drained into dry tubes. The cells were resuspended in alpha MEM medium supplemented with 20% allogeneic plasma and 10ng/ml FGF2 (or with another growth factor known in the art to induce osteoblast phenotype, such as, *e.g.*, BMP). The cells were plated at 1x10⁷ cells/175cm² flasks and maintained in a 37°C humidified atmosphere containing 5% CO2. The cells were allowed to attach for 4 days prior an initial medium change. Two other partial medium changes (half volume changed) are done at days 7 and 11. Cells were detached at day 14 using trypsin-EDTA solution for 1-5 min at 37°C. The cells were counted and plated at 1x10⁶ cells/175cm² for another week of culture.

### B. Bone Marrow mesenchymal cell differentiation in osteogenic medium

Bone Marrow Mesenchymal Stem Cells from standard MSC expansion culture are recovered by incubation with trypsin-EDTA and plated at 60 to 120,000 cells/well in 6-wells plate in the expansion medium (12 500 cells/cm²). The next day, the medium is replaced by 2,5 ml osteogenic medium. The cells are cultured for 2, 3 or 4 weeks. The medium is replaced every 3-4 days.

### Media

Dexamethasone dilution:
Dex1 (5.10⁻⁴M): 2 µl dexamethasone stock (5.10⁻²M) + 198 µl alpha-MEM
Dex2 (10⁻⁶M): 2 µl Dex1 (5.10⁻⁴M) + 998 µl alpha-MEM

### Osteogenic medium (40 ml)

| | Volume | Final concentration |
|---|---|---|
| alpha MEM | 31 ml | / |
| FCS | 6 ml | 15% |
| PenStrepGlu (100x) | 400 µl | 1x |
| Dexamethasone (Dex2) | 400 µl | 10⁻⁸ M |
| Ascorbic acid | 200 µl | 50 µg/ml |
| Beta-glycerophosphate | 2 ml | 10 mM |

### C. Trabecular osteoblasts expansion

From human bone specimen, soft connective and cortical bone were carefully removed, and the remaining trabecular bone was minced into small fragments (1mm²). The bone fragments were extensively washed in PBS to remove the adherent marrow cells and seeded in 25 cm² tissue culture flasks in a culture medium supplemented with autologous serum with or without a growth factor (see above). The medium was changed twice a week. After 4 weeks cells were released using trypsin-EDTA solution, counted and eventually re-plated at a density of 5000 cells/cm².

### Flow cytometry

Immuno-biological cell surface markers of the cells were analyzed by flow cytometry. Bone-forming cells were incubated with the following labelled monoclonal antibodies: HLA-I, HLA-DR, CD80, CD86, CTLA-4, CD40L and CD28 for 15 min and then washed with PBS before being centrifuged and re-suspended in 0.3ml PBS.

OCN was immuno-detected with a specific antibody following fixation and permeabilisation of the cells and the staining was analysed by flow cytometry.

### Mineralization assay

Induction of mineralization potential was assessed by adding osteogenic medium on the cells. -6 000 bone forming cells (see above)/cm² were plated into 6-well plates in presence of 5% autologous plasma supplemented with 0.1 µM dexamethasone, 0.05 mM ascorbic acid and 3 mM glycerol phosphate. After 2, 3 or 4 weeks of culture, cells were fixed in 3.7% formaldehyde/PBS and stained by alizarin red.

### ALP staining

Cells were stained for ALP detection. Bone-forming cells (see above) were washed twice with PBS, then fixed in 60% citrate buffered acetone for 30 seconds at room temperature, and then rinsed again with distilled water for 45 seconds. Cells were then stained with a Fast Blue RR/Naphtol AS-MX phosphate solution for 30 minutes at room temperature, and in the dark. Cells were washed with distilled water for 2 minutes, and then counterstained in Mayer's Hematoxylin solution for 10 minutes. Finally, cells were washed in distilled water for 3 minutes.

### Proliferation assay

200.000 human T cells/ml from individual A (Peripheral Blood Mononuclear Cells, PBMCa) were plated in 96-well microtiter plate with irradiated or CD3 depleted PMBC from individual B (APCb) and human bone-forming cells from a third individual for 10 days in a total volume of 200µl, in presence or not of PHA (a mitogenic activator of T cells). The human bone-forming cells were seeded at 20.000, 100.000 and 200.000/ml. When PBMCa and APCb were coincubated - with or without PHA, a mixed lymphocyte reaction occurred wherein the PBMCa cells were activated by the surface antigens on APCb. The culture was incubated with 1µCi/ml 3H-thymidine for 18h of the culture period to measure T cells proliferation. Cells were washed twice with ice-cold PBS and twice with ice-cold 5% trichloroacetic acid (TCA). Finally, cells were lysed by a solution containing 0.1N NaOH and 0.1% Triton-X100. The supernatant is harvested and mixed with scintillation liquid to be analyzed on a beta-counter.

### Results

### Bone reconstructive properties

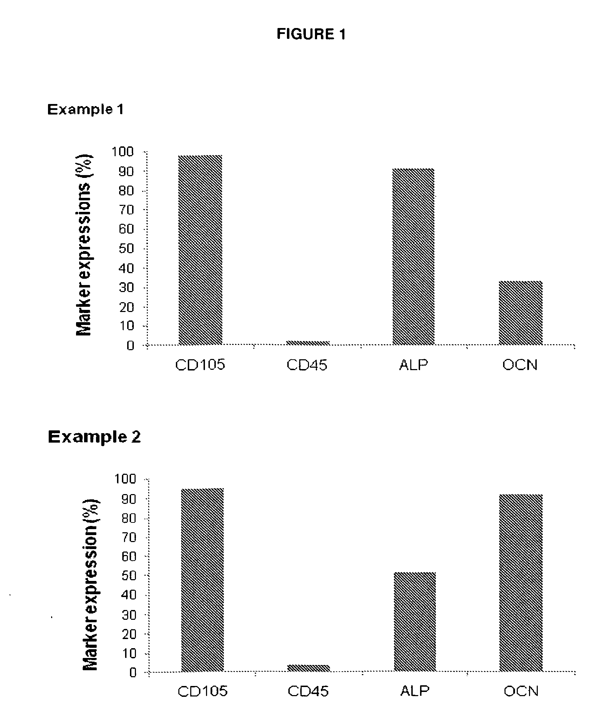

The level of cellular markers (bone and mesenchymal markers) or membrane markers were assessed by flow cytometry after 3 weeks (Figure 1). The bone (ALP, OCN) and mesenchymal (CD105, 73, 90) markers were highly expressed, while hematopoietic marker (CD45) was negative.

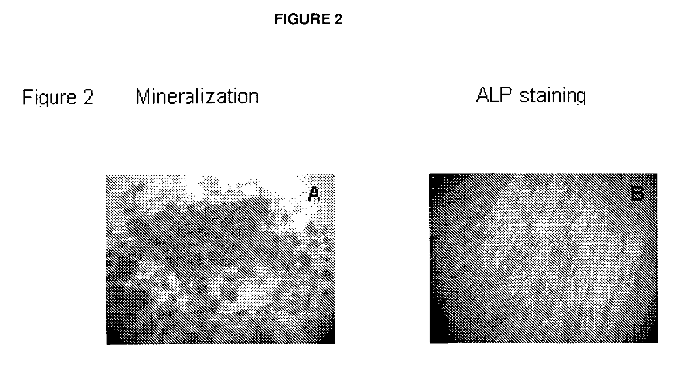

Correlation with bone biological function was done with ALP production (ALP staining) and calcium deposition (mineralization): ALP was expressed by most, if not all cells at day 21 and important mineral deposit - above 65% of total area under microscopic examination - was observed (Figure 2).

### Immuno-modulation properties

Results shown in Tables 1 and 2 indicate that APC were recognized by the PBMC as foreign cells and PBMC were therefore proliferating. When PBMCa and APCb were mixed in the presence of autologous BM derived osteoblasts (from individual B, BMOBb), the mixed lymphocyte reaction was suppressed, by 40-50%.

The same effects were observed in presence of PHA (10µg/ml) which is a strong T cells proliferation stimulator (Table 2). The immunosuppressive effects of osteoblasts were more important - at 55 to 65% - on stimulated T cells.

Interestingly when mixed in the presence allogeneic BM derived osteoblasts (from individual C, BMOBc), the mixed lymphocyte reaction was also significantly suppressed, at 30-40% in standard conditions (Table 1) and 60-65% in PHA stimulated conditions. BM derived osteoblasts from individual C (BMOBc) were loaded on 96-well microtiter plate and coincubated with PBMCa and APCb. The culture was incubated with 3H-thymidine for 18h of the culture period to measure T cell proliferation. These results suggest that there was no specificity of suppression with respect to the HLA subtype.

**Table 1**

| | | | |
|---|---|---|---|
| MSC n° | PBMCa + APCb | PBMCa + APCb + BMOBb | PBMCa + APCb + BMOBc |
| 1 | 15,000 +/- 2,100 | 9, 000 +/- 1,350 | 10,000 +/- 1,500 |
| 2 | 14,000 +/- 1,750 | 7,000 +/- 350 | 9,000 +/- 550 |
| 3 | 17,000 +/- 200 | 12,000 +/- 1,500 | 12,000 +/- 750 |

**Table 2. Inhibitory effects of BM derived osteoblasts (BMOB) on PHA activated T cells proliferation (values are presented in cpm of incorporated [³H]Thimidine)**

| MSC n° | PBMCa + APCb + PHA | PBMCa + APCb + BMOBb + PHA | PBMCa + APCb + BMOBc + PHA |
|---|---|---|---|
| 1 | 25,000 +/- 2,800 | 12,000 +/- 1,700 | 13,000 +/- 1,300 |
| 2 | 22,000 +/- 2,200 | 8,000 +/- 1,300 | 10,000 +/- 950 |
| 3 | 35,000 +/- 3,400 | 15,000 +/- 1,200 | 13,000 +/- 1,100 |

### Conclusions

Bone-forming cells such as osteoprogenitors, pre-osteoblasts or osteoblasts can be useful in treating inflammatory rheumatic disease as they display both bone reconstructive and anti-inflammatory properties. These cells are characterized by high expression levels of mesenchymal and bone surface markers, correlated with ALP enzymatic activity and mineralization ability, confirming their bone biological profile. The cells are further capable to downregulate the proliferative response of stimulated T cells on an autologous and allogeneic basis. This demonstrates that autologous or allogeneic bone marrow derived bone forming cell products can be particularly useful for the treatment of inflammatory rheumatic diseases.

### EXAMPLE 2: Model of in vivo inflammation (IRD)

### Model of Carrageenan-induced ankle/paw inflammation (1)

Inflammation was induced by injection of a solution containing 1% Carrageenan (CARRA; Sigma, Switzerland) in PBS into the hind paw of 8 weeks old SWISS mice; each animal receiving a single injection in each hind paw (Table 3). Carrageenan-injected animals received immediately after injection or PBS only, or a solution of Dexamethasone (DEX; Sigma Switzerland) at a concentration of 1 mg/kg, or 1*10⁶ human bone-forming cells (OB) (derived from bone marrow mesenchymal stromal cells as described in Example 1 A) or a combination of DEX and OB.

**Table 3: experimental protocol**

| Groups (n=4) | Left hind paw | Right hind paw |
|---|---|---|
| #1 | CARRA 0.7% | CARRA 0.7%+ DEX (1 mg/kg) |
| #2 | CARRA 0.7% | CARRA 0.7% + OB 10⁶ |
| #3 | CARRA 0.7% | CARRA 0.7% + DEX + OB |
| #4 | CARRA 0.7% | PBS |

Under isoflurane sedation, the circumference of ankles and paws was measured using a digital caliper before the injection at T0, and at T1, T4 and T24; respectively before CARRA administration, and 1 h, 4h, 6h and 24 hours after the injection.

### Model of Carrageenan-induced ankle/paw inflammation (2)

Inflammation was induced by injection of a solution containing 1% lambda carrageenan (CARRA; Sigma, Switzerland) in PBS into the hind paw of 8 weeks old SWISS mice; each animal receiving a single injection in each hind paw (Table 4). Carrageenan injected animals received immediately after injection or PBS only, or a solution of Dexamethasone (DEX; Sigma Switzerland) at a concentration of 1 mg/kg, or 1*10⁶ human bone-forming cells (OB) (derived from bone marrow mesenchymal stromal cells as described in Example 1 A).

**Table 4: experimental protocol**

| Groups (n=4) | Left hind paw | Right hind paw |
|---|---|---|
| #1 | CARRA 0.7% | CARRA 0.7% + DEX (1mg/kg) |
| #2 | CARRA 0.7% | CARRA 0.7% + OB 10⁶ |
| #3 | CARRA 0.7% | PBS |

Under isoflurane sedation, the circumference of ankles and paws was measured using a digital caliper before the injection at T0 and at T1, T4, T6 and T24; respectively before CARRA administration, and 1 h, 4h, 6h and 24 hours after the injection.

### Model of Adjuvant-induced ankle/paw inflammation

Inflammation was induced by injection of a solution containing 0.5% complete Freund adjuvant (ADJ; Sigma, Switzerland) in PBS into the hind paw of 8 weeks old SWISS mice; each animal receiving a single injection in each hind paw (Table 5). Adjuvant-injected animals received immediately after injection or PBS only, or a solution of Dexamethasone (DEX) at a concentration of 1 mg/kg, or 1*10⁶ human bone-forming cells (OB) (derived from bone marrow mesenchymal stromal cells as described in Example 1 A).

**Table 5: experimental protocol**

| Groups (n=4) | Left hind paw | Right hind paw |
|---|---|---|
| #1 | ADJ 75µg | ADJ 75µg + DEX (1 mg/kg) |
| #2 | ADJ 75µg | ADJ 75µg + OB 10⁶ |
| #2 | ADJ 75µg | PBS |

Under isoflurane sedation, the circumference of ankles and paws was measured using a digital caliper before the injection at T0 and at T1, T4, T6 and T24; respectively before ADJ administration, and 1 h, 4h, 6h and 24 hours after the injection.

### Results: Effects on Carrageenan-induced Inflammation

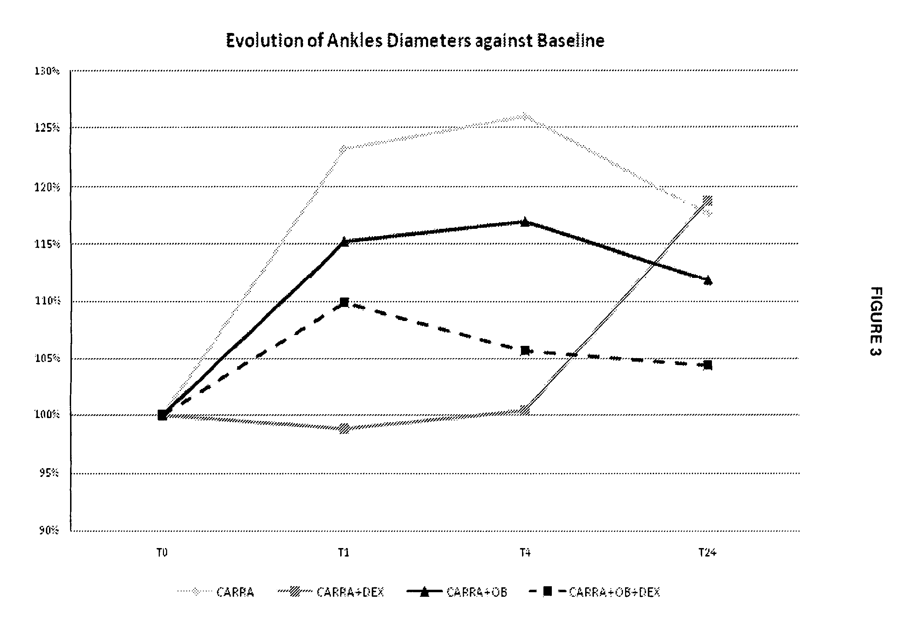

Carrageenan injection induces an immediate inflammatory reaction measurable by the increase in (paw or ankle) diameters on the injected animals. The diameter increase, over baseline, is approximately 20% after 1 hour, 25% after 4 hours and 17% at 24h (Fig. 3).

In the ankle, Dexamethasone (1mg/kg) induces a potent inhibition of the inflammation and the swelling of the paw/ankle injected with Carrageenan (Fig. 3). The inhibition of inflammation induced by dexamethasone is 100% at 1h and 4h but starts to escape thereafter to be totally lost at 24h.

By comparison in the ankle, administration of bone forming cells induces a moderate, but important, inhibition of the inflammation at 1h and 4h, with a decrease of 30% and 40% respectively, but this inhibition seems long lasting with an inhibition maintained at 40% at 24h.

Interestingly, in the carrageenan-induced inflammation, a synergistic (and potent) effect of the combination of bone forming cells and dexamethasone is observed (Fig. 3). The anti-inflammatory effects are 50%, 80 % and 75% at respectively 1, 4 and 24h.

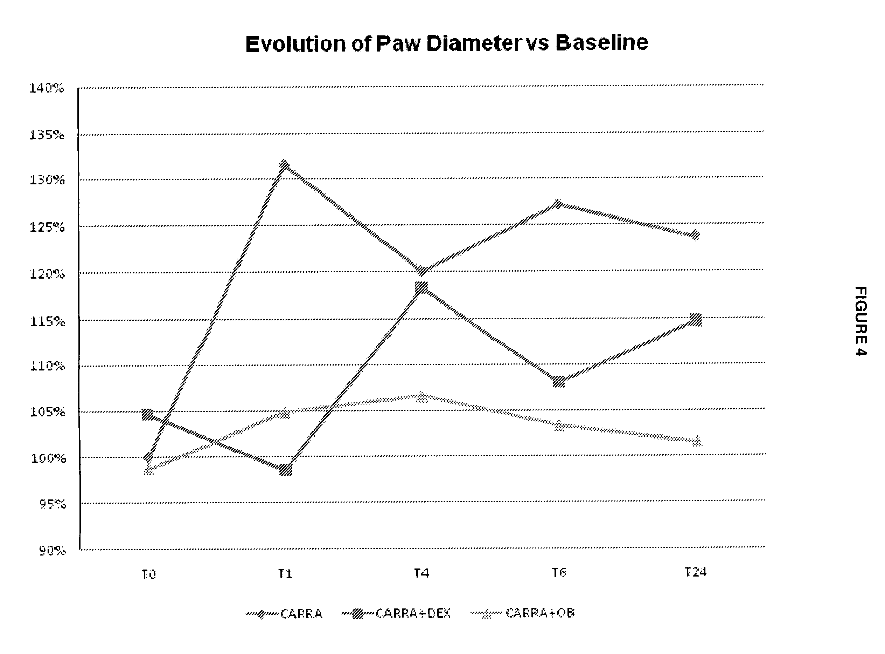

The anti-inflammatory effects for bone-forming cells are stronger, and also long lasting, in the paw. Dexamethasone displays a 100%, 70% and 38% inhibition at 1h, 6h and 24h respectively, against 80%, 90% and 95% for bone forming cells (Fig 4). This may be due to the better distribution and diffusion of injected cells in the paw.

### Results: Effect on adjuvant-induced Inflammation

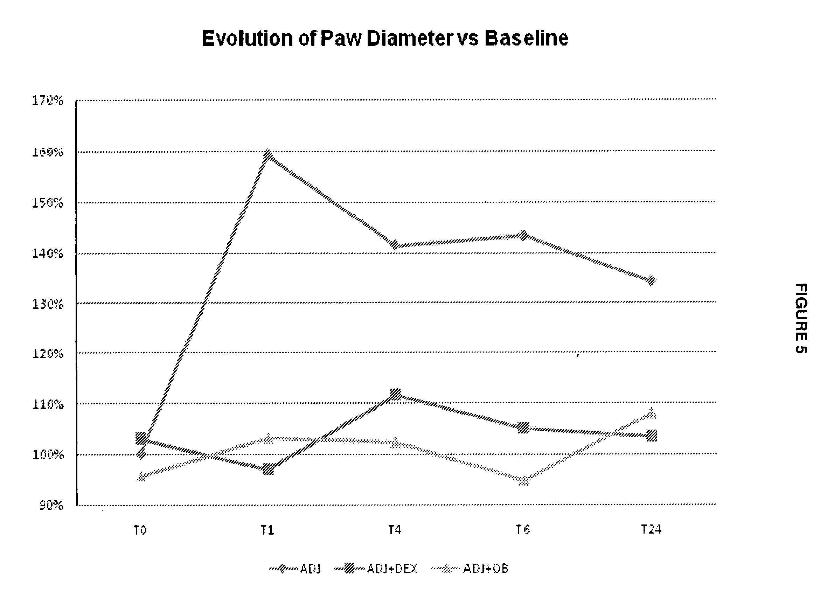

In the adjuvant-induced inflammation, despite a more severe inflammation of the paw/ankles (i.e., a 40-50% increase in diameter versus baseline at 4h to 6h), bone forming cells tend to show a potent anti-inflammatory effects (peak effect of 75-90%) similar to dexamethasone inhibition (Fig. 5), which is maintained at 24h.

## Claims

1. Isolated osteoblasts for use in treating the inflammatory component of inflammatory rheumatic diseases (IRD).

2. Isolated osteoblasts for use in treating the inflammatory component of IRD according to claim 1, wherein said osteoblasts are derived by differentiation from isolated bone marrow stem cells or mesenchymal stem cells.

3. Isolated osteoblasts for use in treating the inflammatory component of IRD according to any of claims 1 or 2 wherein the osteoblasts are of human origin and the medicament is to be employed for autologous or allogeneic administration to human subjects.

4. Isolated osteoblasts for use in treating the inflammatory component if IRD according to claim 3 wherein said administration is systemic, topical, intra-articular or peri-articular.

5. Isolated osteoblasts for use in treating the inflammatory component of IRD according to any of claims 1 to 4 wherein the IRD is chosen from osteoarthritis (OA), psoriatic arthropathy, gout, pseudogout and arthritis of various origins comprising rheumatoid arthritis (RA), enteropathic arthritis, reactive arthritis and Reiter syndrome, osteonecrosis, pauciarticular juvenile rheumatoid arthritis, Still disease, Behçet disease, systemic lupus erythematosus, septic arthritis and spondyloarthropathies comprising ankylosing spondylitis, enteropathic spondylitis and undifferentiated spondyloarthropathy.

6. Isolated osteoblasts for use in treating the inflammatory component of IRD according to any of claims 1 to 4 wherein the IRD is other than osteoarthritis (OA), rheumatoid arthritis (RA) and osteonecrosis.

7. Isolated osteoblasts for use treating the inflammatory compound of IRD according to any of claims 1 to 6 wherein the IRD comprises both an inflammation and bone lesion(s).

8. Isolated osteoblasts for use in treating the inflammatory component of IRD according to any of claims 1 to 6 wherein the IRD comprises an inflammation and does not comprise bone lesion(s).

9. A pharmaceutical composition comprising osteoblasts and an agent chosen from disease-modifying antirheumatic drugs (DMARD) and glucocorticoids, for simultaneous, sequential or separate use in treating the inflammatory component of IRD.

10. A pharmaceutical composition comprising osteoblasts for use in treating the inflammatory component of IRD, particularly for use as defined in any one of claims 2 to 8.

## Patentansprüche

1. Isolierte Osteoblasten zur Verwendung bei der Behandlung der entzündlichen Komponente von entzündlichen rheumatischen Erkrankungen (IRD).

2. Isolierte Osteoblasten zur Verwendung bei der Behandlung der entzündlichen Komponente von IRD nach Anspruch 1, wobei die Osteoblasten aus der Differenzierung von isolierten Knochenmark-Stammzellen oder mesenchymalen Stammzellen stammen.

3. Isolierte Osteoblasten zur Verwendung bei der Behandlung der entzündlichen Komponente von IRD nach einem der Ansprüche 1 oder 2, wobei die Osteoblasten menschlichen Ursprungs sind und das Arzneimittel zur autologen oder allogenen Verabreichung an menschliche Patienten zu verwenden ist.

4. Isolierte Osteoblasten zur Verwendung bei der Behandlung der entzündlichen Komponente von IRD nach Anspruch 3, wobei die Verabreichung systemisch, topisch, intraartikulär oder periartikulär ist.

5. Isolierte Osteoblasten zur Verwendung bei der Behandlung der entzündlichen Komponente von IRD nach einem der Ansprüche 1 bis 4, wobei die IRD ausgewählt ist aus Osteoarthritis (OA), Psoriasis arthropatica, Gicht, Pseudogicht und Arthritis verschiedenen Ursprungs, umfassend rheumatoide Arthritis (RA), enteropathische Arthritis, reaktive Arthritis und Reiter'sche Krankheit, Osteonekrose, nur wenige Gelenke betreffende juvenile rheumatoide Arthritis, Still'sche Krankheit, Behçet-Krankheit, systemischen Lupus Erythematosus, septische Arthritis und Spondyloarthropathien umfassend Spondylitis ankylosans, enteropathische Spondylitis und undifferenzierte Spondyloarthropathie.

6. Isolierte Osteoblasten zur Verwendung bei der Behandlung der entzündlichen Komponente von IRD nach einem der Ansprüche 1 bis 4, wobei die IRD verschieden von Osteoarthritis (OA), rheumatoider Arthritis (RA) und Osteonekrose ist.

7. Isolierte Osteoblasten zur Verwendung bei der Behandlung der entzündlichen Komponente von IRD nach einem der Ansprüche 1 bis 6, wobei die IRD sowohl eine Entzündung als auch Knochenverletzung(en) umfasst.

8. Isolierte Osteoblasten zur Verwendung bei der Behandlung der entzündlichen Komponente von IRD nach einem der Ansprüche 1 bis 6, wobei die IRD eine Entzündung umfasst und keine Knochenverletzung(en) umfasst.

9. Pharmazeutische Zusammensetzung, umfassend Osteoblasten und einen Wirkstoff, ausgewählt aus krankheitsmodifizierenden Antirheumatika (DMARD) und Glucocorticoiden für die gleichzeitige, sequenzielle oder getrennte Verwendung bei der Behandlung der entzündlichen Komponente von IRD.

10. Pharmazeutische Zusammensetzung, umfassend Osteoblasten für die Verwendung bei der Behandlung der entzündlichen Komponente von IRD, insbesondere für die Verwendung wie in einem der Ansprüche 2 bis 8 definiert.

## Revendications

1. Ostéoblastes isolés pour utilisation dans le traitement de la composante inflammatoire des maladies rhumatismales inflammatoires (MRI).

2. Ostéoblastes isolés pour utilisation dans le traitement de la composante inflammatoire des MRI selon la revendication 1, dans lesquels lesdits ostéoblastes sont dérivés par différentiation de cellules souches de moelle osseuse ou de cellules souches mésenchymateuses isolées.

3. Ostéoblastes isolés pour utilisation dans le traitement de la composante inflammatoire des MRI selon l'une quelconque des revendications 1 ou 2, dans lesquels les ostéoblastes sont d'origine humaine et le médicament doit être employé pour une administration autologue ou allogénique à des sujets humains.

4. Ostéoblastes isolés pour utilisation dans le traitement de la composante inflammatoire des MRI selon la revendication 3, dans lesquels ladite administration est systémique, topique, intra articulaire ou péri-articulaire.

5. Ostéoblastes isolés pour utilisation dans le traitement de la composante inflammatoire des MRI selon l'une quelconque des revendications 1 à 4, dans lesquels la MRI est choisie parmi l'arthrose (A), l'arthropathie psoriasique, la goutte, la pseudogoutte et l'arthrite de diverses origines comprenant l'arthrite rhumatoïde (AR), l'arthrite entéropathique, l'arthrite réactionnelle et le syndrome de Reiter, l'ostéonécrose, l'arthrite rhumatoïde juvénile pauci-articulaire, la maladie de Still, la maladie de Behçet, le lupus érythémateux systémique, l'arthrite septique et les spondyloarthropathies comprenant la spondylarthrite ankylosante, la spondylarthrite entéropathique et la spondyloarthropathie indifférenciée.

6. Ostéoblastes isolés pour utilisation dans le traitement de la composante inflammatoire des MRI selon l'une quelconque des revendications 1 à 4, dans lesquels la MRI est autre que l'arthrose (A), l'arthrite rhumatoïde (AR) et l'ostéonécrose.

7. Ostéoblastes isolés pour utilisation dans le traitement de la composante inflammatoire des MRI selon l'une quelconque des revendications 1 à 6, dans lesquels la MRI comprend à la fois une inflammation et un ou des lésions osseuses.

8. Ostéoblastes isolés pour utilisation dans le traitement de la composante inflammatoire des MRI selon l'une quelconque des revendications 1 à 6, dans lesquels la MRI comprend une inflammation et ne comprend pas de lésion(s) osseuse(s).

9. Composition pharmaceutique comprenant des ostéoblastes et un agent choisi parmi les antirhumatismaux modificateurs de la maladie (ARMM) et les glucocorticoïdes, pour une utilisation simultanée, séquentielle ou séparée dans le traitement de la composante inflammatoire d'une MRI.

10. Composition pharmaceutique comprenant des ostéoblastes pour utilisation dans le traitement de la composante inflammatoire des MRI, en particulier pour utilisation telle que définie dans l'une quelconques des revendications 2 à 8.
